# FASCICULE DE BREVET EUROPEEN

(11) **EP 2 037 984 B1**
(45) Date de publication et mention de la délivrance du brevet: **09.08.2017**
(21) Numéro de dépôt: 07825823.3
(22) Date de dépôt: 18.06.2007
(51) Int. Cl.: A61M 5/168

(54) **DISPOSITIF MÉDICAL POUR L'ADMINISTRATION D'UNE SOLUTION**
MEDIZINISCHE VORRICHTUNG ZUR VERABREICHUNG EINER LÖSUNG
MEDICAL DEVICE FOR ADMINISTERING A SOLUTION

(30) Priorité: 06.07.2006 EP 06116727
(43) Date de publication de la demande: 25.03.2009
(60) Demande divisionnaire: 13156640.8
(73) Titulaire: DEBIOTECH S.A., 1004 Lausanne (CH)
(72) Inventeur: NEFTEL, Frédéric, 1005 Lausanne (CH)
(74) Mandataire: Grosfillier, Philippe
(86) Numéro de dépôt international: PCT/IB2007/052329
(87) Numéro de publication internationale: WO 2008/004148

(56) Documents cités:
- WO-A-2005/110515
- US-A- 5 681 285
- US-A1- 2005 277 912

## Description

### Domaine de l'invention

La présente invention se situe dans le domaine de l'administration de solutions médicales, par exemple de solutions contenant de l'insuline.

### Etat de la technique

Il existe plusieurs types de dispositifs médicaux destinés à l'administration d'une solution. De tels dispositifs sont constitués d'au moins un réservoir communiquant avec une pompe, le tout étant relié à un patient au moyen d'une tubulure ou d'un accès sous-cutané, intra-musculaire ou veineux..

La solution qui est contenue dans le réservoir est administrée en général sur une certaine durée ou à intervalles déterminés.

Un problème constaté avec les dispositifs de l'état de la technique résulte du fait que la concentration de la solution peut varier au cours du temps du fait de son stockage dans le réservoir. Voir à cet effet le graphe ci-joint qui montre la variation pendant 35 jours de la concentration d'insuline dans un réservoir de type "Topas" qui comporte sur l'une de ses parois un film souple constitué de surlin à l'intérieur et mylar à l'extérieur, la solution d'insuline étant conservée à 37°C

Les causes de cette variation peuvent être l'évaporation que subit la solution, la stabilité du médicament ou toute altération et/ou modification de son contenu.

Ce phénomène est d'autant plus accentué lorsque les parois du réservoir sont perméables et/ou que la durée d'administration de la solution est longue. L'administration d'une solution dont la concentration et ou les caractéristiques varient au cours du temps peut provoquer des complications, voire une menace pour le traitement ou la santé du patient.

Il existe donc un besoin de remédier aux problèmes précités

### Description de l'invention

La présente invention constitue une amélioration par rapport aux dispositifs de l'état de la technique.

Dans l'invention, la solution du problème précité consiste en un dispositif médical pour l'administration d'une solution qui comprend des moyens de pompage, un réservoir, des moyens de communication entre ledit réservoir et lesdits moyens de pompage, ainsi qu'une ligne patient adaptée pour relier le dispositif médical à un patient. Le dispositif selon l'invention se caractérise par le fait qu'il comprend des moyens pour déterminer les caractéristiques physico-chimiques de la solution en fonction du temps au moyen d'un modèle mathématique qui tient compte d'une modification de la solution au cours du temps en fonction de l'exposition à la température. Selon un mode de réalisation de l'invention, le modèle mathématique tient compte de la perméabilité des parois du réservoir en fonction du temps. Selon un mode de réalisation de l'invention, le dispositif comprend des moyens pour faire varier l'administration de la solution en fonction d'une variation de la concentration de la solution déterminée au moyen d'un modèle mathématique.

Avantageusement, le modèle mathématique peut notamment tenir compte des caractéristiques d'évaporation, d'absorption, d'adsorption et ou toute autre modification de la solution au cours du temps en fonction de l'exposition à la température, à l'humidité, à la pression et à toute autre condition d'utilisation, ainsi qu'éventuellement des conditions de vieillissement et/ou altération du réservoir au cours du temps. Ainsi les caractéristiques de la solution peuvent être calculée en fonction de ce modèle mathématique à tout instant.

Dès lors que les caractéristiques de la solution sont modifiées, il est souhaitable d'en tenir compte dans le mode d'administration de la solution afin d'assurer une administration efficace et sûre.

Il peut notamment s'agir d'une évaporation de l'eau au travers d'une membrane souple constituant le réservoir au cours du temps, qui a pour conséquence d'augmenter la concentration du principe actif dans la solution. Dans ce cas, une absence de correction de la concentration aurait pour effet de surdoser le médicament administré. Le principe de l'invention est de corriger l'administration de la solution afin d'assurer une quantité toujours efficace de principe actif en utilisant le modèle mathématique pour tenir compte de la concentration effective de la solution au cours du temps et adapter le débit en conséquence. Cette évaporation étant souvent fonction de la température, il peut être utile d'intégrer également dans le modèle mathématique l'effet de l'exposition à la température au cours du temps afin d'être à même de prévoir au plus juste la concentration probable de la solution à un instant donné. Pour cela, il est nécessaire de disposer d'un capteur de température, de préférence en continu, le modèle mathématique étant à même d'intégrer le calcul de l'évaporation au cours du temps selon un modèle qui peut être non-linéaire.

Il peut également être utile d'utiliser le modèle mathématique afin de prévoir à tout instant la quantité de certaines substances contenues dans le réservoir, tels que des conservateurs (métacrésol, phénol) très souvent utilisés en association avec certaines substances médicales (insuline par exemple). Dès lors que la teneur dans certaines de ces substances n'est plus suffisante, il peut être utile de prévenir l'utilisateur qu'un re-remplissage du réservoir est nécessaire ou que le réservoir doit être changé. Certaines de ces substances étant très volatiles, elles ont tendance à être les premières à diminuer dans le réservoir (ex : phénol).

Selon un mode de réalisation de l'invention, il peut être utile de mesurer certains paramètres de la solution dont l'évolution peut servir d'indicateur au modèle mathématique afin de corriger le calcul des caractéristiques de la solution. Il peut, par exemple, s'agir de la conductivité de la solution qui est un bon indicateur de l'évaporation de l'eau au cours du temps.

Selon un mode de réalisation de l'invention, l'utilisateur peut lire en continu des informations relatives aux caractéristiques de la solution contenue dans le réservoir. Une telle indication peut, par exemple, être représentative de la qualité de la solution (Excellente, Moyenne, Limite), afin de lui permettre de prévoir un re-remplissage de la solution et/ou changement de réservoir à l'avance. Une telle indication est très différente de la seule indication aujourd'hui disponible sur les dispositifs d'administration, à savoir le volume résiduel de la solution et offre une plus grande sécurité à l'utilisateur.

La présente invention présente plusieurs avantages, en particulier d'assurer à tout moment que la dose administrée au patient est la plus correcte possible, compte tenu des modifications prédictives des caractéristiques de la solution au cours du temps. En outre, elle permet d'assurer que lorsque les conditions d'efficacité et/ou de sécurité ne sont plus assurées, l'utilisateur est avertit et le réservoir est changé ou re-remplis à temps. Enfin elle permet également de prévenir l'utilisateur suffisamment à l'avance d'un re-remplissage ou changement nécessaire du réservoir.

Il peut être également important, dans le cas d'un re-remplissage du réservoir, de tenir compte du volume résiduel éventuel de solution présente au moment du re-remplissage (dans le cas ou cette solution résiduelle n'est pas vidée) afin de corriger les caractéristiques de la nouvelle solution obtenue après re-remplissage en fonction de la dilution réalisée entre l'ancienne et la nouvelle solution, toujours dans le but d'assurer une meilleure efficacité et sécurité dans l'administration effective de la solution au patient.

Le graphe joint à la présente description montre l'évolution de la concentration de différents composants d'une solution d'insuline U100 (Novorapid) au cours du temps dans un réservoir comprenant une partie rigide en Topas 8007S-04 de Topas Advanced Polymers GmbH, et un film souple constitué de Surlyn 1702 (30 µm) à l'intérieur et de Mylar D820 (12 µm) à l'extérieur, les deux matériaux en provenance de la société Dupont et la solution étant maintenue à température constante (37°C) pendant 35 jours.

Les résultats observés dans le temps, en fonction de la température, relativement à la concentration d'insuline et des différents agents conservateurs sont également indiqués dans les tables suivantes pour des conditions différentes d'évaluation:
A) Etude insuline U100 Novorapid dans 3 poches différentes sur 4 semaines à 35°C avec et sans vibration de la poche :

| Perte totale d'eau (%) | | | | | |
|---|---|---|---|---|---|
| Type de poche | **1** | **3** | **4** | Moyenne | écart type |
| Eau (poids total) | 2.132 | 1.985 | 2.116 | | |
| 02.06.2006 | 95% | 95% | 94% | **94%** | 1% |
| 09.06.2006 | 93% | 93% | 91% | **92%** | 1% |
| 16.06.2006 | 91% | 91% | 89% | **90%** | 1% |
| 23.06.2006 | 89% | 89% | 86% | **88%** | 1% |
| | | | | | |
| **Phenol** | | | | | |
| 02.06.2006 | 99% | 97% | 99% | **98%** | 1% |
| 09.06.2006 | 97% | 96% | 101% | **98%** | 3% |
| 16.06.2006 | 98% | 98% | 100% | **99%** | 1% |
| 23.06.2006 | 97% | 97% | 99% | **98%** | 1% |
| | | | | | |
| **m**-**Cresol** | | | | | |
| 02.02.2006 | 87% | 85% | 87% | **86%** | 1% |
| 09.06.2006 | 81% | 80% | 84% | **81%** | 2% |
| 16.06.2006 | 75% | 74% | 78% | **76%** | 2% |
| 23.06.2006 | 68% | 69% | 71% | **69%** | 2% |
| | | | | | |
| **Insulin** | | | | | |
| 02.02.2006 | 103% | 102% | 105% | **103%** | 2% |
| 09.06.2006 | 109% | 106% | 109% | **108%** | 2% |
| 16.06.2006 | 111% | 110% | 114% | **112%** | 2% |
| 23.06.2006 | 119% | 117% | 123% | **120%** | 3% |

B) Etude insuline U100 Novorapid dans 5 poches différentes (A,B, D, E et F) sur 1 à 6 semaines à différentes températures (4°C et 35°C) avec (vibr) et sans (still) vibration de la poche en comparaison avec de l'insuline contenue dans une carpule (catridge) :

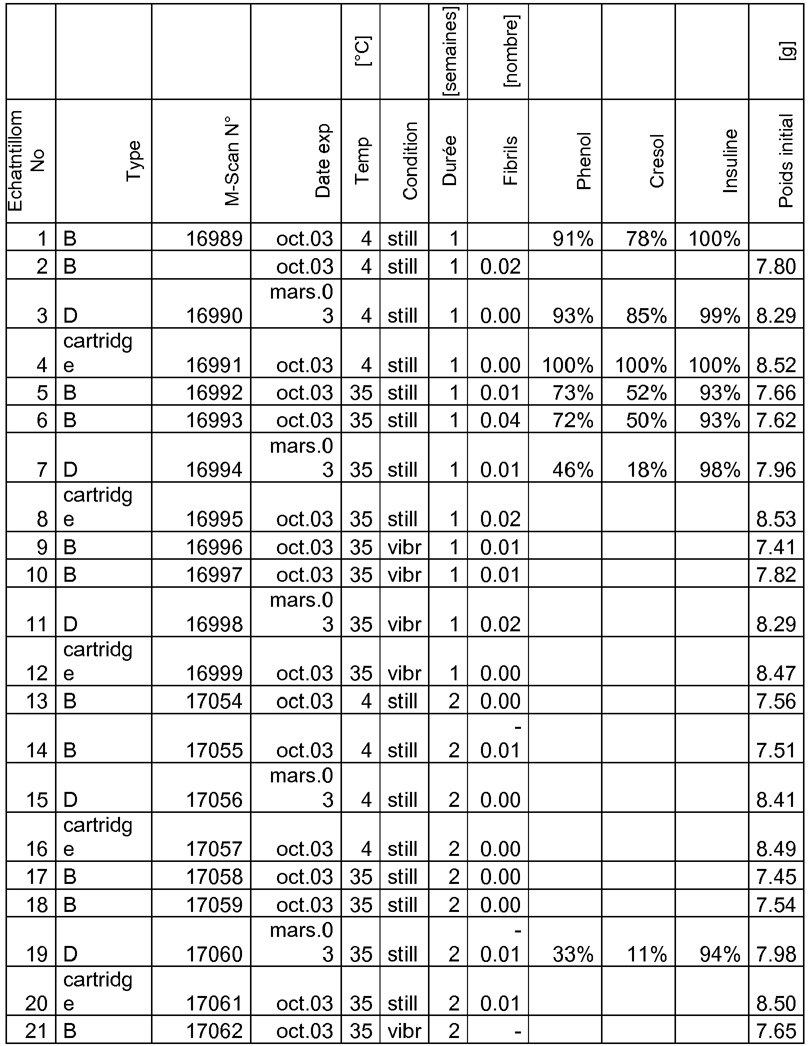

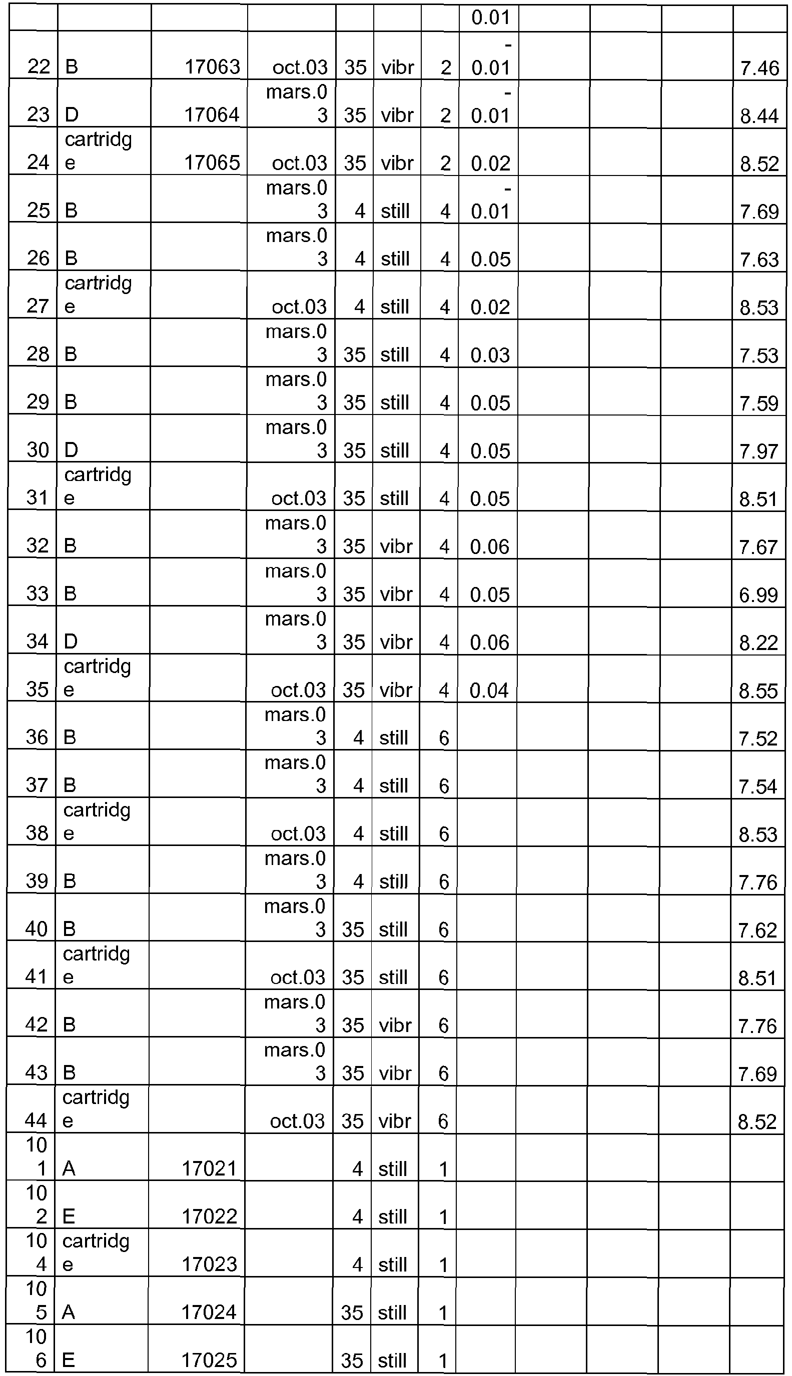

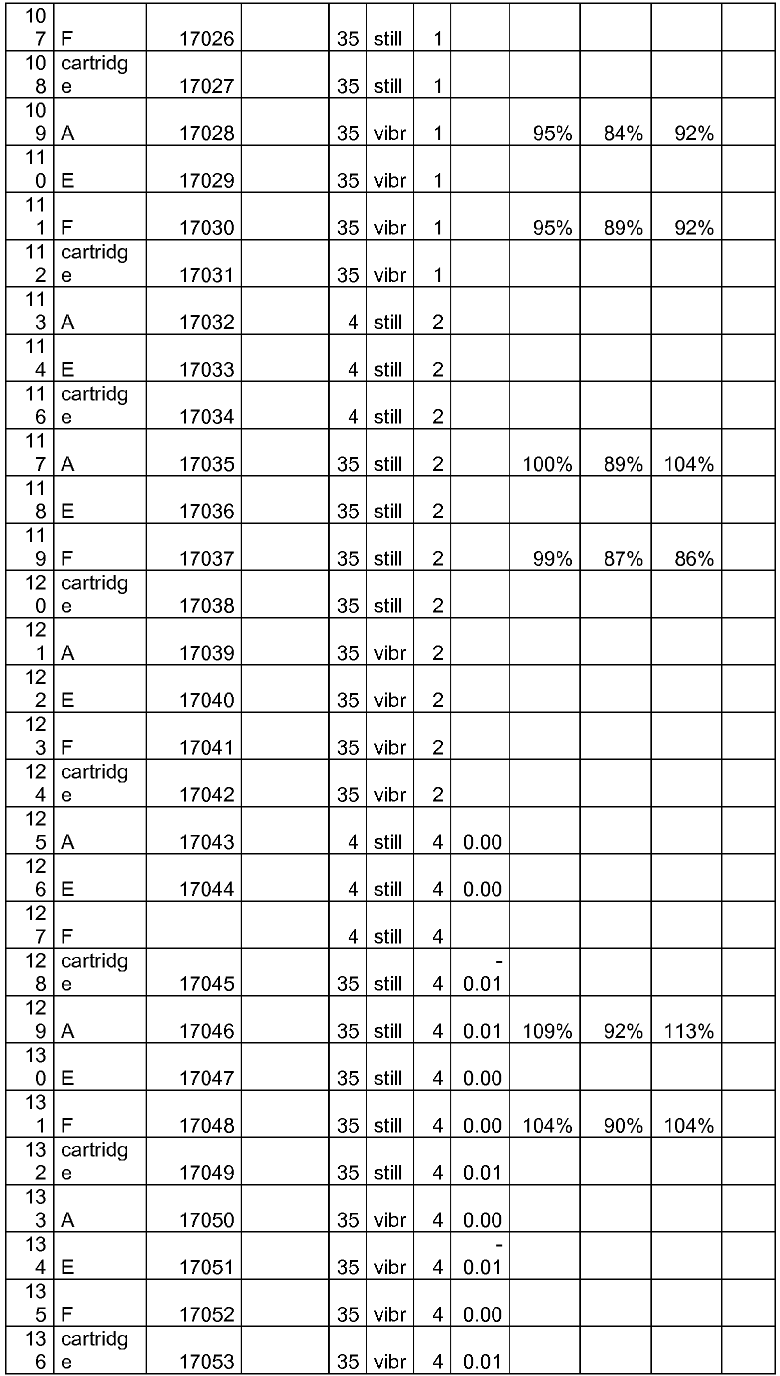

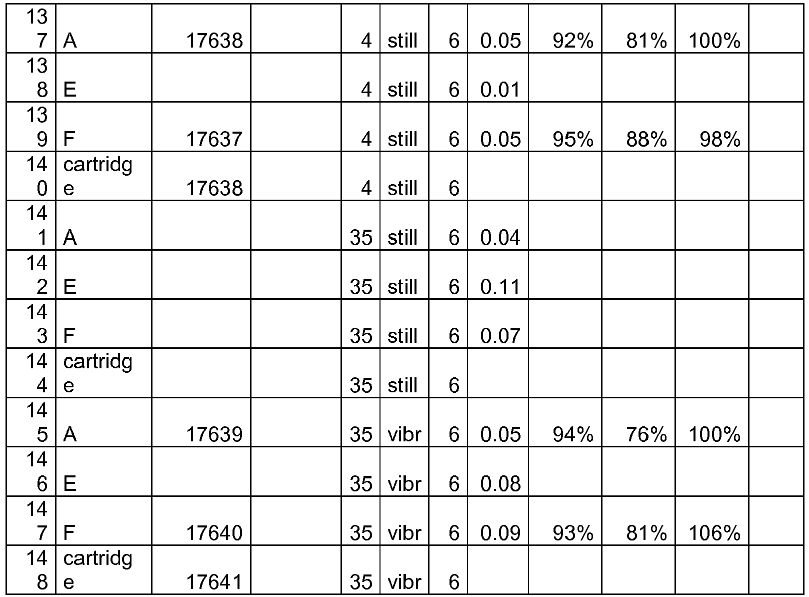

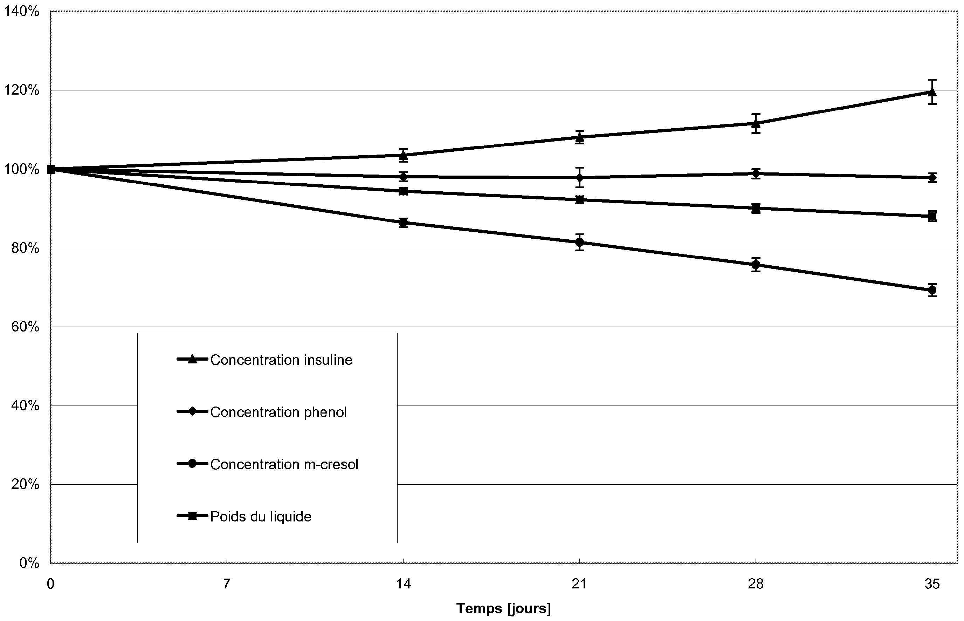

## Revendications

1. Dispositif médical pour l'administration d'une solution, dispositif comprenant des moyens de pompage, un réservoir, des moyens de communication entre ledit réservoir et lesdits moyens de pompage, ainsi qu'une ligne patient adaptée pour relier le dispositif médical à un patient, **caractérisé par le fait qu'**il comprend des moyens pour déterminer les caractéristiques physico-chimiques de la solution en fonction du temps au moyen d'un modèle mathématique qui tient compte d'une modification de la solution au cours du temps en fonction de l'exposition à la température.

2. Dispositif selon la revendication 1 dans lequel le modèle mathématique tient compte de la perméabilité des parois du réservoir en fonction du temps.

3. Dispositif selon l'une des revendications précédentes dans lequel le modèle mathématique tient compte de l'exposition à l'humidité.

4. Dispositif selon l'une des revendications précédentes dans lequel le modèle mathématique tient compte de l'exposition à la pression.

5. Dispositif selon l'une des revendications précédentes dans lequel le modèle mathématique tient compte des conditions de vieillissement et/ou altération du réservoir au cours du temps.

6. Dispositif selon l'une des revendications précédentes dans lequel le modèle mathématique tient compte de l'exposition à toute autre condition d'utilisation.

7. Dispositif selon l'une des revendications précédentes **caractérisé par le fait qu'**il comprend des moyens pour faire varier l'administration de la solution en fonction d'une variation de la concentration de la solution déterminée au moyen du modèle mathématique.

8. Dispositif selon l'une des revendications précédentes comprenant des moyens pour mesurer régulièrement la température de la solution, lesdits moyens pour faire varier l'administration de la solution étant reliés fonctionnellement aux dits moyens pour mesurer en continu la température.

9. Dispositif l'une quelconque des revendications précédentes dans lequel ledit modèle mathématique tient compte de la dégradation de la solution en fonction du temps et/ou de la température.

10. Dispositif selon la revendication 9 , **caractérisé par le fait qu'**il comprend des moyens pour faire varier l'administration de la solution en fonction de la concentration de substance active non-dégradée contenue dans le réservoir.

11. Dispositif selon l'une quelconque des revendications précédentes dans lequel le modèle mathématique est adapté pour déterminer la variation de la nouvelle concentration de la solution après chaque re-remplissage du réservoir en fonction du volume résiduel de la solution et le volume du re-remplissage de la nouvelle solution.

12. Dispositif selon l'une quelconque des revendications précédentes dans lequel le modèle mathématique est adapté pour déterminer les caractéristiques de la solution après chaque re-remplissage du réservoir,

13. Dispositif selon l'une quelconque des revendications précédentes comprenant des capteurs pour mesurer certaines caractéristiques de la solution, lesdits capteurs fournissant une information utilisée par le modèle mathématique.

14. Dispositif selon la revendication 13 dans lequel ledit capteur est un capteur qui mesure la conductivité et/ou la résistance de la solution, le ph ou toute autre mesure physique de la solution.

15. Dispositif selon l'une quelconque des revendications précédentes comprenant une première alarme adaptée pour se déclencher lorsque la solution contenue dans le réservoir est estimée ne plus correspondre aux exigences nécessaires au traitement.

16. Dispositif selon la revendication 15 dans lequel l'alarme a pour but de prévenir l'utilisateur qu'un re-remplissage du réservoir doit être effectué.

17. Dispositif selon l'une quelconque des revendications précédentes comprenant une deuxième alarme adaptée pour se déclencher lorsque la température de la solution contenue dans le réservoir a dépassé un seuil déterminé pendant une durée minimale.

18. Dispositif selon l'une quelconque des revendications précédentes comprenant une troisième alarme adaptée pour se déclencher en fonction d'un modèle mathématique qui tient compte de la température à laquelle est exposée la solution à chaque instant et de la durée de cette exposition.

19. Dispositif selon l'une quelconque des revendications précédentes comprenant un réservoir adapté pour contenir de l'insuline.

20. Dispositif selon l'une quelconque des revendications précédentes dans lequel le réservoir comprend au moins une membrane non totalement imperméable à la vapeur d'eau et/ou à certains composés de la solution.

21. Dispositif selon l'une quelconque des revendications précédentes dans lequel le réservoir et les moyens de pompage sont assurés par une seringue dont les parois ne sont pas totalement imperméable à la vapeur d'eau et/ou à certains composés de la solution.

22. Dispositif selon l'une quelconque des revendications précédentes comprenant des moyens pour indiquer le temps restant d'utilisation possible de la solution dans les conditions d'utilisation courantes et/ou prévisibles.

23. Dispositif selon l'une quelconque des revendications précédentes dans lequel le dispositif médical d'injection est adapté pour informer l'utilisateur de la qualité de la solution selon un mode qui lui permet de prévoir un changement et/ou un re-remplissage à l'avance.

## Patentansprüche

1. Medizinische Vorrichtung für die Verabreichung einer Lösung, wobei die Vorrichtung Pumpmittel, einen Behälter, Kommunikationsmittel zwischen dem Behälter und den Pumpmitteln sowie auch eine Patientenleitung umfasst, die dazu eingerichtet ist, die medizinische Vorrichtung mit einem Patienten zu verbinden, **gekennzeichnet durch** die Tatsache, dass sie Mittel zum Bestimmen der physiko-chemischen Eigenschaften der Lösung in Abhängigkeit von der Zeit mit Hilfe eines mathematischen Modells umfasst, das eine Modifikation der Lösung im Verlaufe der Zeit in Abhängigkeit von der Einwirkung der Temperatur berücksichtigt.

2. Vorrichtung nach Anspruch 1, wobei das mathematische Modell die Durchlässigkeit der Wände des Behälters in Abhängigkeit von der Zeit berücksichtigt.

3. Vorrichtung nach einem der vorhergehenden Ansprüche, wobei das mathematische Modell die Einwirkung der Feuchtigkeit berücksichtigt.

4. Vorrichtung nach einem der vorhergehenden Ansprüche, wobei das mathematische Modell die Einwirkung des Drucks berücksichtigt.

5. Vorrichtung nach einem der vorhergehenden Ansprüche, wobei das mathematische Modell die Alterungsbedingungen und/oder die Veränderung des Behälters im Verlaufe der Zeit berücksichtigt.

6. Vorrichtung nach einem der vorhergehenden Ansprüche, wobei das mathematische Modell die Einwirkung jeder anderen Verwendungsbedingung berücksichtigt.

7. Vorrichtung nach einem der vorhergehenden Ansprüche, **gekennzeichnet durch** die Tatsache, dass sie Mittel umfasst, um die Verabreichung der Lösung in Abhängigkeit von einer Variation der Konzentration der Lösung zu variieren, die mit Hilfe des mathematischen Modells bestimmt wird.

8. Vorrichtung nach einem der vorhergehenden Ansprüche, die Mittel zum regelmäßigen Messen der Temperatur der Lösung umfasst, wobei die Mittel, um die Verabreichung der Lösung zu variieren, funktionsmäßig mit den Mitteln zum kontinuierlichen Messen der Temperatur verbunden sind.

9. Vorrichtung nach irgendeinem der vorhergehenden Ansprüche, wobei das mathematische Modell die Zersetzung der Lösung in Abhängigkeit von der Zeit und/oder der Temperatur berücksichtigt.

10. Vorrichtung nach Anspruch 9, **gekennzeichnet durch** die Tatsache, dass sie Mittel umfasst, um die Verabreichung der Lösung in Abhängigkeit von der Konzentration der nicht zersetzten aktiven Substanz, die in dem Behälter enthalten ist, zu variieren.

11. Vorrichtung nach irgendeinem der vorhergehenden Ansprüche, wobei das mathematische Modell dazu eingerichtet ist, die Variation der neuen Konzentration der Lösung nach jeder Wiederbefüllung des Behälters in Abhängigkeit von dem Restvolumen der Lösung und dem Volumen der Wiederbefüllung der neuen Lösung zu bestimmen.

12. Vorrichtung nach irgendeinem der vorhergehenden Ansprüche, wobei das mathematische Modell dazu eingerichtet ist, die Eigenschaften der Lösung nach jeder Wiederbefüllung des Behälters zu bestimmen.

13. Vorrichtung nach irgendeinem der vorhergehenden Ansprüche, die Sensoren umfasst, um gewisse Eigenschaften der Lösung zu messen, wobei die Sensoren eine Information liefern, die durch das mathematische Modell verwendet wird.

14. Vorrichtung nach Anspruch 13, wobei es sich bei dem Sensor um einen Sensor handelt, welcher die Leitfähigkeit und/oder den Widerstand der Lösung, den Ph oder jedes andere physikalische Maß der Lösung misst.

15. Vorrichtung nach irgendeinem der vorhergehenden Ansprüche, die einen ersten Alarm umfasst, der dazu eingerichtet ist, ausgelöst zu werden, wenn von der Lösung, die in dem Behälter enthalten ist, angenommen wird, dass sie nicht mehr den für die Behandlung notwendigen Anforderungen entspricht.

16. Vorrichtung nach Anspruch 15, wobei der Zweck des Alarms darin besteht, den Benutzer darauf hinzuweisen, dass eine Wiederbefüllung des Behälters durchgeführt werden muss.

17. Vorrichtung nach irgendeinem der vorhergehenden Ansprüche, die einen zweiten Alarm umfasst, der dazu eingerichtet ist, ausgelöst zu werden, wenn die Temperatur der Lösung, die in dem Behälter enthalten ist, einen Schwellenwert überschritten hat, der während einer minimalen Dauer bestimmt wird.

18. Vorrichtung nach irgendeinem der vorhergehenden Ansprüche, die einen dritten Alarm umfasst, der dazu eingerichtet ist, in Abhängigkeit von einem mathematischen Modell ausgelöst zu werden, das die Temperatur, welcher die Lösung zu jedem Zeitpunkt ausgesetzt ist, und die Dauer dieser Einwirkung berücksichtigt.

19. Vorrichtung nach irgendeinem der vorhergehenden Ansprüche, die einen Behälter umfasst, der dazu eingerichtet ist, Insulin zu enthalten.

20. Vorrichtung nach irgendeinem der vorhergehenden Ansprüche, wobei der Behälter mindestens eine Membran umfasst, die nicht vollständig für Wasserdampf und/oder für bestimmte Verbindungen der Lösung undurchlässig ist.

21. Vorrichtung nach irgendeinem der vorhergehenden Ansprüche, wobei der Behälter und die Pumpmittel durch eine Spritze sichergestellt sind, deren Wände nicht vollständig für Wasserdampf und/oder für bestimmte Verbindungen der Lösung undurchlässig sind.

22. Vorrichtung nach irgendeinem der vorhergehenden Ansprüche, die Mittel zum Angeben der möglichen verbleibenden Verwendungszeit der Lösung unter aktuellen und/oder vorhersehbaren Bedingungen umfasst.

23. Vorrichtung nach irgendeinem der vorhergehenden Ansprüche, wobei die medizinische Vorrichtung zur Injektion dazu eingerichtet ist, den Benutzer über die Qualität der Lösung gemäß einem Modus zu informieren, der ihm ermöglicht, im Voraus eine Änderung und/oder eine Wiederbefüllung vorherzusehen.

## Claims

1. Medical device for administering a solution, said device comprising pumping means, a reservoir, means of communication between said reservoir and said pumping means, and a line for connecting the medical device to a patient, **characterized in that** it comprises means for determining the physical/ chemical characteristics of the solution as a function of time by means of a mathematical model that takes account of a modification in the solution over the course of time as a function of the exposure to temperature.

2. Device according to Claim 1, in which the mathematical model takes account of the permeability of the reservoir walls as a function of time.

3. Device according to either of the preceding claims, in which the mathematical model takes account of the exposure to humidity.

4. Device according to one of the preceding claims, in which the mathematical model takes account of the exposure to pressure.

5. Device according to one of the preceding claims, in which the mathematical model takes account of the ageing and/or deterioration of the reservoir over the course of time.

6. Device according to one of the preceding claims, in which the mathematical model takes account of the exposure to any other condition of use.

7. Device according to one of the preceding claims, **characterized in that** it comprises means for varying the administration of the solution as a function of a variation in the concentration of the solution determined by means of the mathematical model.

8. Device according to one of the preceding claims, comprising means for regularly measuring the temperature of the solution, said means for varying the administration of the solution being functionally connected to said means for continuously measuring the temperature.

9. Device according to any one of the preceding claims, in which said mathematical model takes account of the degradation of the solution as a function of time and/or temperature.

10. Device according to Claim 9, **characterized in that** it comprises means for varying the administration of the solution as a function of the concentration of non-degraded active substance contained in the reservoir.

11. Device according to any one of the preceding claims, in which the mathematical model is designed to determine the variation in the new concentration of the solution after each refilling of the reservoir as a function of the residual volume of the solution and the refilling volume of the new solution.

12. Device according to any one of the preceding claims, in which the mathematical model is designed to determine the characteristics of the solution after each refilling of the reservoir.

13. Device according to any one of the preceding claims, comprising sensors for measuring certain characteristics of the solution, said sensors supplying information used by the mathematical model.

14. Device according to Claim 13, in which said sensor is a sensor that measures the conductivity and/or resistance of the solution, the pH or any other physical parameter of the solution.

15. Device according to any one of the preceding claims, comprising a first alarm designed to go off when the solution contained in the reservoir is estimated to no longer correspond to the treatment requirements.

16. Device according to Claim 15, in which the purpose of the alarm is to alert the user to the fact that the reservoir has to be refilled.

17. Device according to any one of the preceding claims, comprising a second alarm designed to go off when the temperature of the solution contained in the reservoir has exceeded a defined threshold during a minimal duration.

18. Device according to any one of the preceding claims, comprising a third alarm designed to go off as a function of a mathematical model that takes account of the temperature to which the solution is exposed at each instant and also of the duration of this exposure.

19. Device according to any one of the preceding claims, comprising a reservoir designed to contain insulin.

20. Device according to any one of the preceding claims, in which the reservoir comprises at least one membrane not totally impermeable to water vapour and/or to certain components of the solution.

21. Device according to any one of the preceding claims, in which the reservoir and the pumping means are provided by a syringe whose walls are not totally impermeable to water vapour and/or to certain compounds of the solution.

22. Device according to any one of the preceding claims, comprising means for indicating the time remaining for possible use of the solution under the current and/or foreseeable conditions of use.

23. Device according to any one of the preceding claims, in which the medical injection device is designed to inform the user of the quality of the solution in a way allowing said user to anticipate a change and/or refilling in advance.
